# EUROPEAN PATENT APPLICATION

(11) **EP 4 759 260 A1**
(43) Date of publication of application: **17.06.2026**
(21) Application number: 24219199.7
(22) Date of filing: 11.12.2024
(51) Int. Cl.: A61B 34/00, A61B 90/00, A61B 18/00, A61B 17/00, A61B 18/12, A61B 17/32, A61M 13/00, A61B 18/08

(54) **CONTROL SYSTEM FOR CONTROLLING A SURGICAL WORKSTATION COMPRISING AN ENERGY-BASED MEDICAL DEVICE**

(71) Applicant: OLYMPUS SURGICAL TECHNOLOGIES EUROPE Olympus Winter & Ibe GmbH, 22045 Hamburg (DE)
(72) Inventor: WOLTER, Michael, 22045 Hamburg (DE)
(74) Representative: Weickmann & Weickmann PartmbB

(57) **Abstract**

The present invention provides a control system for controlling a surgical workstation 10, comprising an energy controller 14 for controlling energy supply of an energy-based medical device 12 for energy treatment of an object, and an auxiliary controller 20, 24, 28 for controlling operation of at least one auxiliary medical device (18, 22, 26; 118, 122, 126) for interaction with said object on the basis of a different modality than the energy-based medical device.

## Description

The present invention relates to control system and method for controlling a surgical workstation, the workstation comprising an energy controller for controlling energy supplied to an energy-based medical device for energy treatment of an object, and an auxiliary controller for controlling operation of at least one auxiliary medical device for interaction with the object on a basis of a different modality than the energy-based medical device.

Energy-based medical devices are advanced devices integral to modern surgical and therapeutic practices, utilizing various forms of energy or various modalities, such as electrical, thermal, laser, high-frequency or ultrasonic modalities, to interact with and treat patient tissue. These devices are employed for a range of applications including cutting, coagulating, ablating or stimulating tissue with high precision and minimal invasiveness. Examples include electrosurgical units, laser units, ultrasonic scalpels, and radiofrequency operation devices.

Energy-based medical devices are often integrated into advanced surgical workstations so as to work together with auxiliary devices such as imaging devices, robotic systems, insufflators, smoke management devices etc. For example, if an energy-based treatment is to be performed in a minimal invasive procedure such as laparoscopy, insufflators are used to deliver a controlled flow of gas, typically carbon dioxide (CO₂), to inflate body cavities and create a clear working space for the surgeons. Furthermore, to remove surgical smoke generated by operation of the energy-based medical device, surgeons may operate smoke evacuators included in the surgical workstation. Smoke evacuators comprise high-efficiency filtration systems to capture airborne particles, toxic gases, and odors, ensuring a clear surgical field and protecting the health of operating room personnel.

In addition, imaging devices included in surgical workstations are used during surgical procedures to provide real-time visualization of internal structures to enhance precision and safety of the surgical procedure. Typical imaging modalities comprise visual-light imaging using cameras, grey-scale imaging, infrared imaging, CT imaging (and BI), X-ray imaging, ultrasound imaging, CT imaging or MRI imaging.

When surgeons are focused on the manipulation and operation of an energy-based medical device during a surgical procedure in order to perform a certain treatment with precision and safety, it can be demanding to operate auxiliary devices such as smoke management devices, imaging devices or insufflators in coordination with operating the energy-based medical device. In order to assist surgeons with those multiple tasks, auxiliary controllers for controlling the auxiliary medical devices are continuously improved to facilitate quick and intuitive operation by the surgeon or by medical assistance personnel. However, coordinating multiple auxiliary medical devices for optimum support of the actual treatment using the energy-based medical device remains a challenging task.

It is therefore an object of the present invention to provide a control system and method for controlling a surgical workstation which address the above-mentioned topics and assist surgeons in operating auxiliary medical devices during an energy-based medical treatment.

According to a first aspect of the present invention, the above-mentioned object is achieved by a control system for controlling a surgical workstation, comprising at least one energy controller for controlling energy supply of an energy-based medical device for energy treatment of an object, at least one auxiliary controller for controlling at least one auxiliary medical device of a different modality than the at least one energy-based medical device (at least one auxiliary medical device for interaction with said object on the basis of a different modality than the energy-based medical device), and a control processor, wherein the control processor is configured to coordinate operations of the energy controller and the auxiliary controller based on operational conditions of the respective devices (in particular the energy-based medical device(s) and/or the auxiliary medical device(s)). In particular, the control processor may be configured to control operation of one of the energy controller and the auxiliary controller based on a detected operational condition of the other one of the energy controller and the auxiliary controller.

According to an important feature of the first aspect of the invention, a control processor is provided which is connected to both the energy controller and the auxiliary controller. Therefore, in one embodiment, the control processor may be adapted to control operation of the energy controller based on a detected operational condition of the auxiliary controller. In another embodiment, the control processor (or another control processor) may be adapted to control operation of the auxiliary controller based on a detected operational condition of the energy controller. In other words, a particular operation performed by the energy-based medical device may automatically trigger a particular operation of the at least one auxiliary medical device and/or a particular operation of the at least one auxiliary medical device may automatically trigger a particular operation of the energy-based medical device. In this way, at least some of the control operations usually to be performed by the surgeon or the medical assistance personnel can be carried out by the control processor according to the first aspect of the present invention such as to assist the operating room team in performing the surgical procedure and allow the surgeon to better focus on the actual energy-based treatment.

In a preferred embodiment of the present invention, the control processor may be adapted to control operation of the auxiliary controller based on at least one of the following detected operational conditions of the energy controller: Switch-on or switch-off of energy delivery, a pulse parameter of a pulse-type energy delivery, and an amplitude value of the energy currently delivered. In particular, switching on or off energy delivery may result in a modification or reaction of the object or any other change within the surgical space around the object. The control system of the present embodiment then automatically triggers a corresponding operation of the at least one auxiliary medical device. For example, switching on energy delivery of an energy-based cutting or ablation device may result in generation of surgical smoke, and the control processor may then control an operation of a smoke evacuation device (auxiliary medical device) in order to evacuate such smoke without or with reduced need of manual interaction by the surgeon or the medical assistance personnel.

If the energy-based medical device uses a pulse-type energy delivery, the detected operational condition of the energy controller may refer to a pulse parameter of the pulse type energy delivery such as the type of the pulse (square, sawtooth, sinus, etc.), pulse duration, pulse on/off relationship or duty cycle, a pulse frequency, a pulse power etc. In other embodiments, the detected operational condition of the energy controller may refer to an amplitude of the energy currently delivered. The above-mentioned operational conditions of the energy controller are usually commanded by the surgeon while handling the energy-based medical device and performing a certain treatment.

In addition, or alternatively, the control processor can be adapted according to control, based on a detected operational condition of the auxiliary controller, at least one of the following operations of the energy controller: switching on or switching off energy delivery, setting a pulse parameter of pulse-type energy delivery, setting an amplitude of the energy to be delivered. In this way, detected operational conditions of the auxiliary controller, including operational conditions of the at least one auxiliary medical device as well as sensor data detected by the auxiliary medical device, may be used to trigger automatic changes in the settings or the actual operation of the energy-based medical device. For example, if the auxiliary medical device comprises a smoke analysis device which detects smoke characteristics indicating suboptimal settings of the energy-based medical device, the control processor may automatically change - by controlling operation of the energy controller - the settings of the energy-based medical device, for example to reduce or even stop energy delivery or optimize the energy settings.

In an embodiment of the present invention, the energy controller may be adapted to fully control, as the energy-based medical device, at least one of a high-frequency device, a laser device, and an ultrasonic device. Such devices are particularly useful to be operated in cooperation and in coordination with auxiliary medical devices. However, other forms of energy or other modalities may be used by the energy-based medical device to achieve a certain therapeutic effect on a patient's tissue, for example thermal energy, radio frequency or microwave energy, e.g. for thermal ablation or coagulation, mechanical energy such as shockwaves, light-based energy or cryogenic modalities. In the sense of the present invention, the energy-based medical device is operating based on any of the above modalities to deliver energy for a particular treatment. On the other hand, the at least one auxiliary medical device is operating based on any modality different from the modality used above by the energy-based medical device and is used to assist the actual treatment of the patient's tissue as performed by the surgeon using the energy-based medical device.

In another embodiment of the present invention, the at least one auxiliary medical device can comprise a gas management device. The gas management device can be an insufflator for use in minimal invasive procedures such as laparoscopy to deliver a controlled flow of gas, typically carbon dioxide (CO₂) to inflate body cavities and create a clear working space for the surgeon. Insufflators need to maintain precise pressure and flowrate levels. The generation of surgical smoke during an energy-based treatment therefore requires re-adjustment of operational parameters of the insufflator. Consequently, in an embodiment of the present invention, operating the insufflator by the control processor based on detected operational conditions of the energy controller (and thus the energy-based medical device) may greatly facilitate the coordination to the energy-based treatment and the operation of the insufflator.

In a further embodiment of the invention, the gas management device (auxiliary medical device) can comprise a smoke evacuation device, wherein the control processor can be adapted to control, based on a detected operational condition of the energy controller, at least one of the following operations of the smoke evacuation device: start suction operation of the smoke evacuation device, stop suction operation of the smoke evacuation device, and change flow rate of the smoke evacuation device. Thus, not only can a suction operation to evacuate surgical smoke be initiated automatically upon operation of the energy-based medical device to relief the surgeon from manipulating the smoke evacuator, but also can the operation of the smoke evacuation device be more precisely adjusted and adapted with respect to timing and flow rate (suction capacity) in conformity with the actual operation of the energy-based medical device.

In a further embodiment of the present invention, the gas management device can comprise a smoke evacuation device for evacuating smoke from a side of the object, and a smoke analysis device adapted to detect at least one smoke characteristic of smoke evacuated by the smoke evacuation device, wherein the control processor can be adapted to control operation of the energy controller based on the detected at least one smoke characteristic. Suitable detectors provided in the smoke analysis device can detect at least one of: a content of water or liquid within the smoke, a particle density, a size of smoke particles or a chemical composition of smoke particles. Furthermore, the smoke analysis device may observe a time dependency of the above-mentioned smoke characteristics. By controlling the energy controller through the control processor based on such smoke characteristics, particular settings, operational modes or other parameters of the energy-based medical device may be adjusted or otherwise controlled to ensure a safe and precise treatment.

In a further embodiment of the present invention, the at least one auxiliary medical device may comprise an imaging device adapted to capture an image of the object. The imaging device may use different types of imaging techniques or imaging modalities such as visible light imaging, grey-scale imaging, infrared imaging, narrow band imaging (NBI), X-ray imaging, ultrasound imaging, CT imaging or MRI. Based on a detected operational condition of the energy controller, the control processor may then control the operation of the imaging device in order to react to possible changes of the object or the space around the object, to continuously ensure optimum visualization of the object and the surgical space for the surgeon. For example, the control processor may change the imaging modality or an imaging wavelength of the imaging device in response to the generation of surgical smoke that emerges upon operation of the energy-based medical device. The above-described embodiment of the present invention allows an automatic triggering of such change in control of the imaging device. In addition or alternatively, the image captured by the imaging device may be corrected based on a detected operational condition of the energy controller for example if the control processor takes into account that certain image artifacts are originating from a reaction of the object to the energy-based treatment, for example surgical smoke generated as a result of the treatment.

In addition or alternatively, the control processor may be adapted to control operation of the energy controller based on an image detected by the imaging device. In particular, from an image analysis of an image captured by the imaging device, the control processor may conclude that the energy-based medical device is operated using suboptimal operational parameters or suboptimal operating conditions. Operating the energy controller based on the detected image may then automatically optimize operation of the energy-based medical device and therefore increase precision and safety of the treatment.

In a preferred embodiment of the present invention, the control processor may comprise a machine learning system. In particular, the machine learning system may comprise a neural network, for example, a convolutional neural network (CNN), which can be operative to generate output data for operating one of the energy controller and the auxiliary controller based on input data indicating a detected operational condition of the other one of the energy controller and the auxiliary controller.

In an embodiment of the invention, the machine learning system may be trained to optimize energy usage patterns of the energy-based medical device based on detected operational conditions of at least one auxiliary device. Such optimization may be performed in real-time, thus allowing continuous operation.

For training such machine learning system as well as, more generally, for training any machine learning system for use with a surgical workstation comprising an energy-based medical device and at least one auxiliary medical device, according to a second aspect of the present invention, for solving the above mention objective problem, there is provided a method comprising inputting a plurality of training datasets into a machine learning system, wherein each training dataset comprises: energy data representing a particular operation of an energy-based medical device for energy-treatment of an object, and auxiliary data representing a particular operation of at least one auxiliary medical device for interaction with said object on the basis of a different modality than the energy-based medical device, wherein said particular operation of the energy-based medical device is associated to the particular operation of the energy-based medical device.

Each training dataset may be obtained from historical data reflecting actual operations carried out on a surgical workstation, by a surgeon who actively controls the energy-based medical device for a particular treatment and who further controls at least one auxiliary medical device in a manner suitable for the particular situation and the particular treatment, i.e., in association with the particular operation of the energy-based medical device. Therefore, each training dataset may reflect control operations, including settings, control commands and detected values of the devices, for a particular action or a particular situation during a surgical treatment.

In addition, or alternatively, the training datasets may include energy data and/or auxiliary data which are partially or completely based on theoretical data defined by a surgeon or another person skilled in the art as being suitable for a particular situation during a surgical treatment.

In addition, or alternatively, the training datasets may comprise weighted training datasets, wherein each training dataset comprises energy data, auxiliary data associated to these energy data, and a weighting factor indicating an evaluation of a correlation between the energy data and the corresponding auxiliary data. The weighting factor may reflect a range of evaluation values ranging from low or poor evaluation values, if the energy-based medical device and the at least one auxiliary medical device, when operated according to the energy data and the auxiliary data, show poor coordination with one another and operate in a manner regarded not sufficient for the particular situation from a viewpoint of a surgeon, to high or excellent evaluation values, if the energy-based medical device and the at least one auxiliary medical device, when operated according to the energy data and the auxiliary data, show perfect coordination such as to master the current surgical situation and perform their individual tasks in an optimal manner from a view point of a surgeon.

After having trained a machine learning system using a method as described above using a sufficient number of training datasets, the machine learning system is able to predict output data based on unseen input data, i.e., generate energy data based on unseen auxiliary data, and/or generate auxiliary data based on unseen energy data. Generated energy data and/or auxiliary data may then be used by the control processor to control operation of the energy controller and/or the auxiliary controller, respectively.

According to a third aspect of the present invention, the above-mentioned object is achieved by a surgical workstation comprising an energy-based medical device, at least one auxiliary medical device, and a control system according to the first aspect of the present invention connected to the energy-based medical device and the at least one auxiliary medical device. In this manner a surgical workstation is provided which can achieve the same or corresponding features and effects as described above as the first or second aspect of the invention.

According to a fourth aspect of the present invention, the above-mentioned objective problem is achieved by a method for controlling a surgical workstation, the method comprising: controlling energy supply to an energy-based medical device performing energy-treatment of an object based on a detected operational condition of at least one auxiliary medical device interacting with said object on the basis of a different modality than the energy-based medical device, or controlling, based on a detected operational condition of an energy-based medical device performing energy-treatment of an object, an operation of at least one auxiliary medical device interacting with said object on the basis of a different modality than the energy-based medical device. By a method of the fourth aspect of the present invention, the same or corresponding features and effects can be achieved as described above for the first aspect of the present invention. In particular, a method according to the fourth aspect of the present invention may be carried out using a control system according to the first aspect of the present invention.

According to an embodiment of the method of the fourth aspect, the detected operational condition may comprise at least one of: switch on or switch off energy delivery, a pulse parameter of a pulse-type energy delivery and an amplitude value of the energy currently delivered.

In a further embodiment of the fourth aspect of the invention, controlling energy supply to the energy-based medical device may comprise at least one of switching on or switching off energy delivery, setting a pulse parameter of a pulse-type energy delivery, and setting an amplitude of the energy to be delivered.

The at least one auxiliary medical device may comprise a gas management device. In particular, the gas management device may comprise a smoke evacuation device, wherein the method of the fourth aspect of the present invention may perform, based on a detected operational condition of the energy-based medical device, at least one of the following operations of the smoke evacuation device: start or stop a suction operation, and change a flow rate of the smoke evacuation device. In an embodiment of the fourth aspect of the invention, operation of the energy controller may be controlled based on at least one smoke characteristic detected for smoke evacuated from a side of the object.

In a further embodiment of the fourth aspect of the invention, an operation of an imaging device may be controlled based on a detected operational condition of the energy-based medical device, and/or at least one image captured by an imaging device may be corrected based on a detected operational condition of the energy-based medical device. In addition, or alternatively, an operation of the energy-based medical device can be controlled based on an image detected by the imaging device.

In embodiments of the fourth aspect of the present invention, a control of the energy-based medical device based on a detected operational condition of the at least one auxiliary device may comprise processing data within a machine learning system. In addition or alternatively, control of at least one auxiliary medical device based on a detected operational condition of the energy-based medical device may comprise processing data within a machine learning system. The machine learning system in both cases preferably comprises a neural network, more particularly a trained neural network, for example a convolutional neural network (CNN).

In a further embodiment of the present invention, training of the machine learning system can be carried out or continued during operation of the control system during an actual surgical procedure by implementing at least one of the following steps:
- feeding back a detected operational condition (including settings, control parameters, command values, sensor values, etc.) of the energy-based medical device or the energy controller to the machine learning system,
- feeding back a detected operational condition (including settings, control parameters, command values, sensor values, etc.) of the at least one auxiliary medical device or the auxiliary controller to the machine learning system,
- obtaining a weighting factor or an evaluation value indicating the quality of a match between a currently detected operational condition of the energy-based medical device or the energy controller on the one hand and a currently detected operational condition of the auxiliary medical device or the auxiliary controller on the other hand, (in particular, indicating a degree of coordination between an operation of the energy-based medical device and an operation of the at least one auxiliary medical device), and inputting the weighting factor or evaluation value into the machine learning system,
- feeding back data from at least one sensor, for example, a pressure sensor, a temperature sensor, a humidity sensor, a sensor detecting a wavelength of an emitted spectrum, etc.

Preferred embodiments of the present invention will now further be described in connection with the attached drawings, in which:
- Figure 1: shows a schematic drawing of the structure of a surgical workstation according to a first embodiment of the present invention,
- Figures 2a, 2b, and 2c: are diagrams showing some examples of time dependent operational conditions of an energy-based medical device and a smoke evacuation device in a surgical workstation according to the first embodiment,
- Figure 3: shows a schematic drawing of the structure of a surgical workstation according to a second embodiment of the present invention, and
- Figure 4: shows a smoke analysis device of the surgical workstation according to the second embodiment.

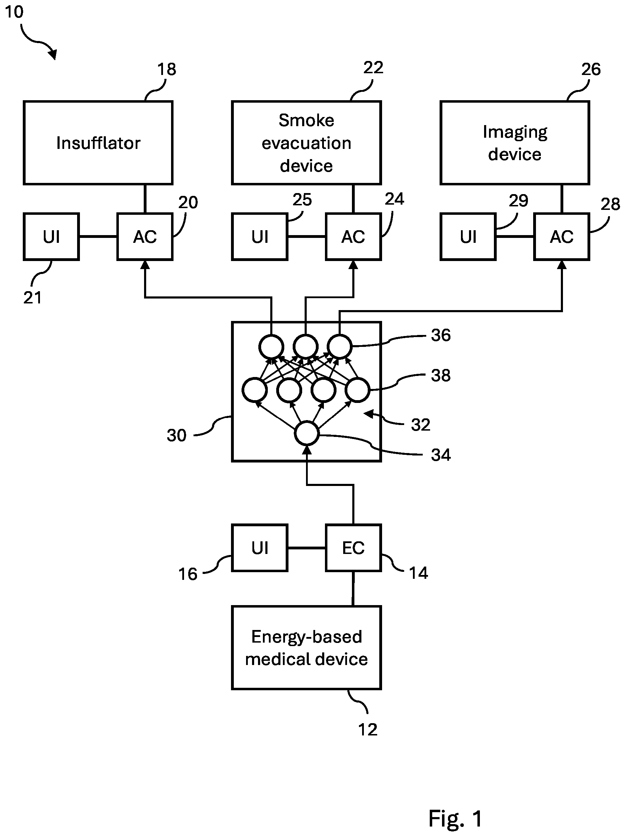

A surgical workstation 10 according to first embodiment of the present invention is shown in a schematic way in figure 1 and comprises an energy-based medical device 12, such as a high-frequency treatment device, a laser device or an ultrasonic device for performing an energy-based treatment of a patient's tissue, for example cutting, ablating or stimulating tissue during a laparoscopic or open surgery. Energy-based medical device 12 is connected to an energy controller 14 which controls energy supply of the energy-based medical device 12, i. e., to start or stop an energy supply or to change setting or an operational mode or to detect or sense a current operational mode or operational condition of the energy-based medical device 12.

Energy controller 14 is further connected to a user interface 16 allowing a user, i. e., a surgeon, to input control values, to start or stop energy supply or adjust operational parameters of the energy-based medical device 12, for example a pulse width, a pulse duration, a duty cycle or another pulse parameter of a pulse-type energy supply, or an amplitude of energy to be supplied. The energy-based medical device 12 may be a suitable hand-held tool to be manipulated by the surgeon, while the user interface 16 may be implemented by or comprise suitable buttons or other control elements provided directly at the hand-held tool or at any other position at the surgical workstation that can be conveniently reached and operated by the surgeon or medical assistant personnel.

The surgical workstation 10 of the first embodiment may further comprise an insufflator for controlling a flow of gas such as carbon dioxide into a body cavity. Operation of the insufflator may be controlled, monitored and analyzed by an insufflator controller (auxiliary controller) 20 connected to the insufflator 18. Furthermore, a user interface 21 can be connected to the insufflator controller 20 in order to allow operating room personnel to directly control operation of the insufflator 18, i.e., start or stop the flow of gas, adjust gas flow rate, or read out an operation condition of the insufflator 18.

Surgical workstation 10 can further comprise a smoke evacuation device 22 for sucking or evacuating surgical smoke from a side of the object in order to ensure a clear surgical field and protecting the health of the operating room personnel. Operation of the smoke evacuation device 22 may be controlled or monitored through a smoke controller 24 connected to the smoke evacuation device 22. A user interface 25 may be connected to the smoke controller 24 which is accessible for direct operation by the surgeon and/or the medical assistance personnel in order to control or monitor operation of the smoke evacuation device 22.

In addition, the surgical workstation 10 may comprise an imaging device 26 providing real-time visualization of internal structures during a surgical procedure based on a suitable imaging modality, such as visual light imaging, infrared imaging, narrow band imaging, grey scale imaging, x-ray imaging, ultrasound imaging, CT imaging or MRI. An imaging controller 28 can be connected to the imaging device 26 to allow operating room personnel to control an operation of the imaging device 26, i.e., start or stop an imaging process, set or change imaging parameters such as an imaging wavelength, focus or a camera field of view etc. An imaging controller 28 connected to the imaging device 26 may be configured to receive, process and analyze images captured by the imaging device 26 in order to enable presentation of the images to the operating room personnel. For example, imaging controller 28 may be connected to display. Furthermore, a user interface 29 my be connected to imaging controller 28 to allow operating room personnel to actively control or monitor operation and/or settings of the imaging device 26.

In the sense of the present invention, insufflator devise 18, smoke evacuation device 22 and imaging devise 26 are regarded as auxiliary devices assisting the operation of the energy-based medical device 12, the latter being used for the actual treatment of the patient's tissue. Accordingly, insufflator controller 20, smoke controller 24 and imaging controller 28 are each regarded auxiliary controllers in the sense of the present invention.

Surgical workstation 10 according to the first embodiment further comprises a control processor 30 connected on one side to the auxiliary controllers such as the insufflator controller 20, the smoke control 24 and the imaging controller 28, and on the other side to the energy controller 14. Control processor 30 may comprise a machine learning system in the form of a neural network 32 having an input layer 34, an output layer 36 and one or more hidden layers 38 between the input layer 34 and the output layer 36. In the first embodiment, the input layer 34 is connected to and receives input data from the energy controller 14, while the output layer 36 is connected to and outputs data to each of the auxiliary controllers 20, 24, 28. Thus, in addition to or as an alternative to controlling auxiliary devices 18, 22 and 26 directly by the operating room personnel through the respective user interfaces 21, 25 or 29, respectively, the auxiliary devices 18, 22 and 26, according to the first embodiment of the present inventio, are controlled based on output data provided by the neural network 32 of the control processor 30 in response to the input data received from the energy controller 14.

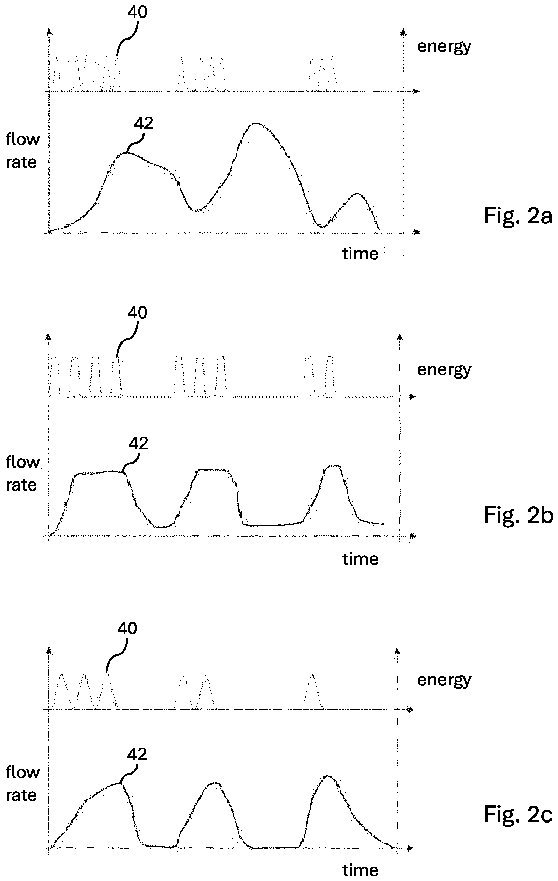

Figures 2a, 2b and 2c show three examples of operations of the surgical workstation according to the first embodiment. The diagrams of figures 2a, 2b and 2c each show a time dependency of the energy data of 40 provided by energy controller 14 (input data to control processor 30), i.e., data reflecting energy supply of the energy-based medical device 12. In addition, the diagrams of figures 2a, 2b and 2c each show a time dependency of flow-rate data 42 (output data of control processor 30) indicating a time dependent flow rate of a suction pump of the smoke evacuation device 22. Flow-rata data 42 are thus output by the neural network 32 in response to the energy data 40.

As can be seen by comparing the figures 2a, 2b and 2c the neural network 32 proposes different flow-rate data 42 for different energy data 40, i.e., proposes to modify the time dependent flow-rate data 42 upon recognizing a change in the energy data 40. Provided sufficient training of the neural network 32 by a sufficient number of qualified training data, the surgical workstation is then able to generate optimal control parameter for optimum control of the auxiliary devices 18, 22 and 26 and/or to automatically trigger operation of the auxiliary devices 18, 22 and 26.

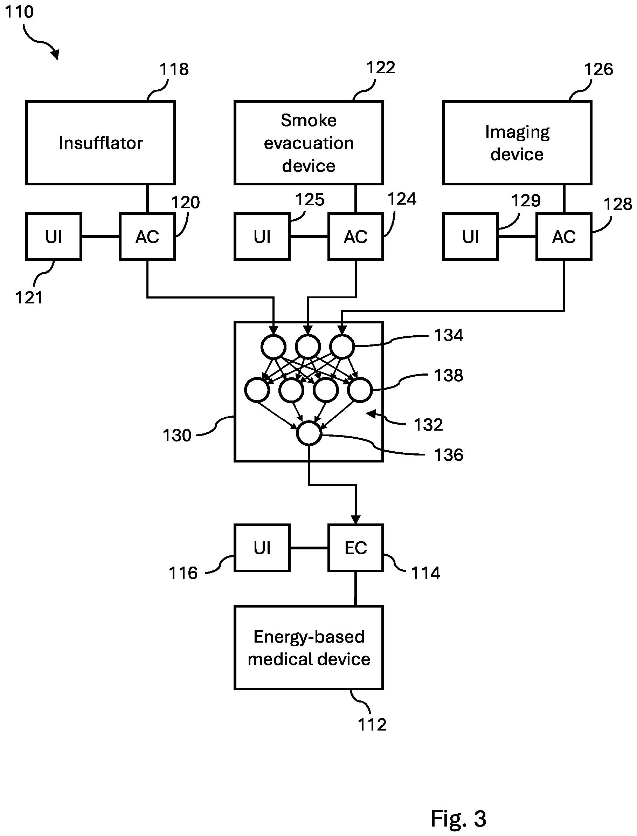

Figure 3 shows a schematic drawing of the structure of a surgical workstation 110 according to a second embodiment on the present invention. The second embodiment is in many aspects identical or analogue to the first embodiment such that reference is made to the more detailed description of the first embodiment above for the same or corresponding components, features and effects of the second embodiment.

In particular surgical workstation 110 of the second embodiment of the invention comprises an energy-based medical device 112 connected to an energy controller 114 which in turn may be connected to a user interface 116 for allowing operating room personnel to operate the energy-based medical device 112 through energy control 114. The configuration and functions of the energy-based medical device 112, energy controller 114, and user interface 116 may be the same or corresponding to those described above with respect to the first embodiment.

Surgical workstation 110 may further comprise auxiliary devices such as an insufflator 118, a smoke evacuation device 122 and an imaging device 126.

Each of the auxiliary devices may be connected to respective auxiliary controllers 120, 124, 128, in the same or corresponding manner and with the same or corresponding functions as described above for the first embodiment. Furthermore, user interfaces 121, 125, and 129 may be provided individually for each auxiliary controller 120, 124 and 128, respectively, in order to allow operating room personnel to input control commands into or read out operational conditions from the respective auxiliary controllers 120, 124, 128, and, effectively into or from the respective auxiliary devices 118, 122, and 126, respectively.

The surgical workstation 110 of the second embodiment comprises a control processor 130 which includes a neural network 132 having an input layer 134, an output layer 136, and one or more hidden layers 138 between the input layer 134 and the output layer 136. As a difference to the first embodiment described above, in the second embodiment of the present invention as shown in figure 3, the auxiliary controllers 120, 124, and 128 of the respective auxiliary devices 118, 122, and 126 are respectively connected to the input layer 134 of the neural network 132, and the energy controller 114 of the energy-based medical device 112 is connected to the output layer 136 of the neural network 132. Consequently, the neural network 132 of the second embodiment is trained and configured to receive, as input data, auxiliary data indicating operational conditions (including settings, current operational parameters, detected sensor values, command values, etc.) from at least one of the auxiliary devices 118, 122 and 126, and to generate, as output data, energy data to be supplied to the energy controller 114 for controlling operation of the energy-based medical device 112. The generated energy data may be used to change settings or change an operational mode of the energy-based medical device 12, for example to minimize carbonization, adjust a speed of cutting or ablation etc.

The auxiliary data input into input layer 134 of neural network 132 may for example comprise information about smoke generated during the energy-based treatment, wherein such smoke information may be provided either by the smoke controller 124 based on an operational condition or a sensed value from the smoke evacuation device 122, or by the imaging controller 128 based on an analysis of images captured by imaging device 126.

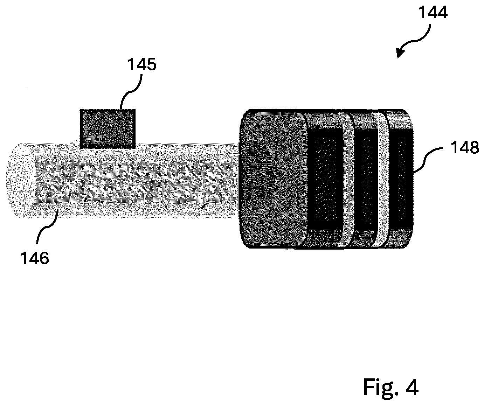

The smoke evacuation device 122 and/or the insufflator 118 can further comprise a smoke analysis device 144. As shown more specifically in figure 4, smoke analysis device 144 may comprise a suction channel 146 and one or more filters 148, as conventionally known as such, wherein a suction pump (not shown in the drawings) may be operated to generate a flow of smoke from the object side through suction channel 146 and then through filters 148. A sensor 145 may be arranged within or attached to suction channel 146 (or filters 148) in order to detect or sense a smoke characteristic of the surgical smoke.

The at least one or more characteristic detected in this manner may in particular comprise information regarding at least one of a content of water or liquid components in the smoke, a particle density of smoke particles or a time dependency of the particle density, a chemical composition of smoke particles, and a size of smoke particles. Such smoke characteristic can be input as auxiliary data into the neural network 132 which then generates energy data as output data based on which the energy controller 14 then controls the energy-based medical device 112. For example, based on a particular composition or other characteristics of surgical smoke detected by the smoke detector 144, energy settings or control settings of energy-based medical device 12 may be adjusted to minimize complications, reduce speed of cutting or ablation, or otherwise improve precision or safety of the treatment.

## Claims

1. Control system for controlling a surgical workstation (10; 110), comprising
a. at least one energy controller (14; 114) for controlling energy supply of an energy-based medical device (12; 112) for energy treatment of an object,
b. at least one auxiliary controller (20, 24, 28; 120, 124, 128) for controlling at least one auxiliary medical device (18, 22, 26; 118, 122, 126) of a different modality than the at least one energy-based medical device, and
c. a control processor (30; 130), wherein the control processor is configured to coordinate operations of the energy controller and the auxiliary controller based on operational conditions of the respective devices.

2. Control system of claim 1, wherein the control processor (30) is adapted to control operation of the auxiliary controller (20, 24, 28) based on at least one of the following detected operational conditions of the energy controller (14):
- Switch-on or switch-off of energy delivery,
- a pulse parameter of a pulse-type energy delivery,
- an amplitude value of the energy currently delivered.

3. Control system of claim 1, wherein the control processor (130) is adapted to control, based on a detected operational condition of the auxiliary controller (120, 124, 128), at least one of the following operations of the energy controller (114):
- switching on or switching off energy delivery,
- setting a pulse parameter of pulse-type energy delivery,
- setting an amplitude of the energy to be delivered.

4. Control system of at least one of the preceding claims, wherein the energy controller is adapted for controlling, as the energy-based medical device (12; 122), at least one of a high-frequency treatment device, a laser device, and an ultrasonic device.

5. Control system of at least one of the preceding claims, wherein the at least one auxiliary medical device comprises a gas management device.

6. Control system of claim 5, wherein the gas management device comprises a smoke evacuation device (22; 122), and wherein the control processor (30; 130) is adapted to control, based on a detected operational condition of the energy controller, at least one of the following operations of the smoke evacuation device:
- start suction operation of the smoke evacuation device,
- stop suction operation of the smoke evacuation device,
- change flow rate of the smoke evacuation device.

7. Control system of claim 5 or claim 6, wherein the gas management device comprises a smoke evacuation device (22; 122) for evacuating smoke from a side of the object, and a smoke analysis device (144) adapted to detect at least one smoke characteristic of smoke evacuated by the smoke evacuation device,
and wherein the control processor is adapted to control operation of the energy controller based on the detected at least one smoke characteristic.

8. Control system of claim at least one of the preceding claims, wherein the at least one auxiliary medical device comprises an imaging device (26; 126) adapted to capture an image of the object.

9. Control system of claim 8, wherein the control processor (30) is adapted to control operation of the imaging device or to correct at least one image captured by the imaging device, based on a operational condition of the energy controller (14).

10. Control system of claim 8 or claim 9, wherein the control processor (130) is adapted to control operation of the energy controller (114) based on an image detected by the imaging device (126).

11. Control system of at least one of the preceding claims, wherein the control processor comprises a machine learning system (32; 132), wherein the machine learning system is preferably trained to optimize energy usage patterns based on detected operational conditions of auxiliary devices, more preferably in real-time.

12. Surgical workstation (10; 110) comprising an energy-based medical device, at least one auxiliary medical device, and a control system according to at least one of the preceding claims connected to the energy-based medical device and the at least one auxiliary medical device.

13. Method for controlling a surgical workstation, the method comprising:
- controlling energy supply to an energy-based medical device performing energy-treatment of an object based on a detected operational condition of at least one auxiliary medical device interacting with said object on the basis of a different modality than the energy-based medical device, or
- controlling, based on a detected operational condition of an energy-based medical device performing energy-treatment of an object, an operation of at least one auxiliary medical device interacting with said object on the basis of a different modality than the energy-based medical device.

14. Method of claim 13 using a control system according to at least one of the claims 1 to 12.

15. Method for training a machine learning system, comprising inputting a plurality of training datasets into the machine learning system, each training dataset comprising:
- energy data representing a particular operation of an energy-based medical device for energy-treatment of an object,
- auxiliary data representing a particular operation of at least one auxiliary medical device for interaction with said object on the basis of a different modality than the energy-based medical device, wherein said particular operation of the energy-based medical device is associated to the particular operation of the energy-based medical device.
